# EUROPEAN PATENT APPLICATION

(11) **EP 3 591 038 A1**
(43) Date of publication of application: **08.01.2020**
(21) Application number: 18760998.7
(22) Date of filing: 22.02.2018
(51) Int. Cl.: C12N 5/0775, A61K 35/28, A61P 43/00

(54) **MESENCHYMAL STEM CELLS AND PHARMACEUTICAL COMPOSITION**

(30) Priority: 03.03.2017 JP 2017040807
(71) Applicant: Rohto Pharmaceutical Co., Ltd., Osaka-shi Osaka 544-8666 (JP)
(72) Inventor: UENO Yui, Osaka-shi Osaka 544-8666 (JP); NONAKA Hidenori, Osaka-shi Osaka 544-8666 (JP)
(74) Representative: Mewburn Ellis LLP
(86) International application number: PCT/JP2018/006364
(87) International publication number: WO 2018/159432

(57) **Abstract**

The purpose of the present invention is to provide a mesenchymal stem cell that is highly safe when used as a medicine. The present invention is a mesenchymal stem cell characterized in that the Tissue Factor (TF) is expressed at low levels. Furthermore, the present invention includes a mesenchymal stem cell characterized in that at least one integrin gene selected from the group consisting of ITGA11, ITGA1, ITGB5, ITGBL1, ITGB1 and ITGAV is expressed at low levels. Furthermore, the present invention includes a mesenchymal stem cell characterized in that at least one integrin gene selected from the group consisting of ITGA4, ITGA9, ITGA7, and ITGA10 is expressed at high levels. It is preferable that the mesenchymal stem cell is allogeneic and is derived from a fatty tissue. The present invention also includes a pharmaceutical composition containing the mesenchymal stem cell.

## Description

### TECHNICAL FIELD

The present invention relates to mesenchymal stem cells and a pharmaceutical composition.

Mesenchymal stem cells are precursor cells having pluripotency and isolated for the first time from the bone marrow by Friedenstein (1982) (Non Patent Document 1). It has been found that mesenchymal stem cells are present in various tissues including the bone marrow, umbilical cord and adipose, and transplantation of mesenchymal stem cells has been expected as a novel therapy for various intractable diseases (see, Patent Documents 1 and 2). Recently, it has been found that the stromal cells of an adipose tissue, placenta, umbilical cord, vitelline coat, etc. have the same function as that of the mesenchymal stem cells. Because of this, mesenchymal stem cells are sometimes referred to as mesenchymal stromal cells.

However, in the studies for intravenously injecting mesenchymal stem cells into mice, cases where mice died of respiratory failure conceivably caused by impaired circulation in the lung have been reported (see Non Patent Documents 2 to 4). In the circumstance, development of highly safe mesenchymal stem cells is desired.

### PRIOR ART DOCUMENTS

### Patent Documents

Patent Document 1: JP 2012-157263 A
Patent Document 2: JP 2012-508733 A

### Non Patent Documents

Non Patent Document 1: Pittenger F. M. et al., Science, 284, pp. 143-147, 1999
Non Patent Document 2: Furlani D.et al., Microvasc. Res., 77, pp. 370-376, 2009
Non Patent Document 3: Tatsumi K. et al., Biochemical and Biophysical Research Communications, 431, pp. 203-209, 2013
Non Patent Document 4: Shiratsuki S. et al., Hepatology Research, 45, pp. 1353-1359, 2015

### SUMMARY OF INVENTION

### Technical Problem

In the aforementioned circumstances, the present invention aims to provide highly safe mesenchymal stem cells as a medicine.

### Solution to Problem

The present inventors conducted intensive studies with a view to attaining the aforementioned object. As a result, they found that highly safe mesenchymal stem cells can be obtained by culturing mesenchymal stem (stromal) cells (MSCs) in a serum-free medium. Based on the finding, the present invention was accomplished. According to the present invention, it is possible to provide highly safe mesenchymal stem cells as a medicine. More specifically, the present invention is summarized as follows.
[1] Mesenchymal stem cells wherein the expression of Tissue Factor (TF) is low.
[2] Mesenchymal stem cells wherein the expression of at least one integrin gene selected from the group consisting of ITGA11, ITGA1, ITGB5, ITGBL1, ITGB1 and ITGAV is low.
[3] Mesenchymal stem cells wherein the expression of at least one integrin gene selected from the group consisting of ITGA4, ITGA9, ITGA7 and ITGA10 is high.
[4] The mesenchymal stem cells according to any one of [1] to [3], wherein the mesenchymal stem cells are allogeneic.
[5] The mesenchymal stem cells according to any one of [1] to [4], wherein the mesenchymal stem cells are derived from an adipose tissue.
[6] A pharmaceutical composition containing the mesenchymal stem cells according to any one of [1] to [5].
[7] A method for treating diseases comprising a step of using mesenchymal stem cells of which the expression of Tissue Factor (TF) is low.
[8] A method for treating diseases, comprising a step of using mesenchymal stem cells of which the expression of at least one integrin gene selected from the group consisting of ITGA11, ITGA1, ITGB5, ITGBL1, ITGB1 and ITGAV is low.
[9] A method for treating diseases, comprising a step of using mesenchymal stem cells of which the expression of at least one integrin gene selected from the group consisting of ITGA4, ITGA9, ITGA7 and ITGA10 is high.
[10] The method for treating diseases according to any one of [7] to [9], wherein the mesenchymal stem cells are allogeneic.
[11] The method for treating diseases according to any one of [7] to [10], wherein the mesenchymal stem cells are derived from adipose tissue.

### Advantageous Effects of Invention

According to the present invention, it is possible to provide highly safe mesenchymal stem cells for use in a medicine.

### BRIEF DESCRIPTION OF DRAWINGS

[Fig. 1] Fig. 1 shows graphs showing the size (diameter) of the mesenchymal stem cells of the present invention.
[Fig. 2] Fig. 2 is a graph showing mRNA expression of TF in the mesenchymal stem cells of the present invention.
[Fig. 3] Fig. 3 is a graph showing expression of a cell-surface protein of TF in the mesenchymal stem cells of the present invention by FACS analysis.
[Fig. 4] Fig. 4 is a graph showing mRNA expression of ITGB5 in the mesenchymal stem cells of the present invention.
[Fig. 5] Fig. 5 is a graph showing mRNA expression of ITGB1 in the mesenchymal stem cells of the present invention.
[Fig. 6] Fig. 6 is a graph showing mRNA expression of ITGA1 in the mesenchymal stem cells of the present invention.
[Fig. 7] Fig. 7 is a graph showing mRNA expression of ITGA1 1 in the mesenchymal stem cells of the present invention.
[Fig. 8] Fig. 8 is a graph showing mRNA expression of ITGAV in the mesenchymal stem cells of the present invention.
[Fig. 9] Fig. 9 is a graph showing mRNA expression of ITGA4 in the mesenchymal stem cells of the present invention.
[Fig. 10] Fig. 10 is a graph showing mRNA expression of ITGB1 in the mesenchymal stem cells of the present invention.
[Fig. 11] Fig. 11 is a graph showing mRNA expression of ITGA1 in the mesenchymal stem cells of the present invention.
[Fig. 12] Fig. 12 is a graph showing mRNA expression of ITGA4 in the mesenchymal stem cells of the present invention.
[Fig. 13] Fig. 13 is a graph showing mRNA expression of ITGA11 in the mesenchymal stem cells of the present invention.
[Fig. 14] Fig. 14 is a graph showing mRNA expression of ITGAV in the mesenchymal stem cells of the present invention.
[Fig. 15] Fig. 15 is a graph showing mRNA expression of ITGB5 in the mesenchymal stem cells of the present invention.

### DESCRIPTION OF EMBODIMENTS

The mesenchymal stem cells of the present invention and a pharmaceutical composition containing the cells will be described in detail.

### [Mesenchymal stem cells and a pharmaceutical composition containing the mesenchymal stem cells]

The mesenchymal stem cells of the present invention are characterized in that the expression of Tissue Factor (hereinafter also referred to as "TF") is low.

The Tissue Factor (TF) is a glycoprotein of 47kD consisting of 295 amino acids and induced to express on the surface of systemic vascular endothelial cells and monocytes by a mediator such as TNF, IL-1, an acute phase protein, thrombin and endotoxin, or a damage (local factor) of a tissue rich in TF such as a brain, a lung and a placenta. TF is known to be involved in initiation of physiologically extrinsic coagulation by binding to F. VII and activating it.

The phrase "the expression of TF is low" includes a case where the mRNA expression of TF in cells is low; the expression of TF protein is low; or both expressions are low. The mesenchymal stem cells of the present invention are satisfactory if the expression of TF is low compared to other cells; more specifically, the mesenchymal stem cells of the present invention are satisfactory if the expression of TF is low compared to mesenchymal stem cells obtained under conventional conditions of culture (for example, culture carried out in 10% FBS-containing Minimum Essential Medium α (MEMα medium)). The expression of TF is preferably 90% or less, more preferably 80% or less, further preferably 70% or less and particularly preferably 60% or less, compared to that in mesenchymal stem cells obtained under conventional culture conditions.

The mesenchymal stem cells of the present invention are characterized in that expression of at least one integrin gene selected from the group consisting of ITGA11, ITGA1, ITGB5, ITGBL1, ITGB1 and ITGAV is low. The mesenchymal stem cells of the present invention are further characterized in that expression of at least one integrin gene selected from the group consisting of ITGA4, ITGA9, ITGA7 and ITGA10 is high.

Integrin is known as a cell-surface protein, which interacts with an extracellular matrix and transmits an intracellular signal regarding shape and movement of cells and progression of the cell cycle, in response to information of the extracellular matrix.

The phrase "expression of an integrin related gene is low or high" includes the meaning that expression of mRNA of the integrin related gene is low or high in a cell; expression of the protein of the integrin-related gene is low or high; or both of the expressions are low or high. The mesenchymal stem cells of the present invention are satisfactory if expression of an integrin related gene is low or high, compared to other cells. More specifically, the mesenchymal stem cells of the present invention are satisfactory if expression of the integrin related gene is low or high compared to mesenchymal stem cells obtained under conventional conditions of culture (for example, culture carried out in 10% FBS-containing Minimum Essential Medium α (MEMα medium)).

The phrase "expression of the integrin related gene is low" means that the expression of the integrin related gene is preferably 90% or less, more preferably 80% or less, further preferably 70% or less and particularly preferably 60% or less, compared to that in mesenchymal stem cells obtained under conventional culture conditions. More specifically, the phrase "expression of ITGA1 is low" means that expression of ITGA1 is preferably 50% or less, more preferably 40% or less, further preferably 30% or less and particularly preferably 20% or less, compared to that in the mesenchymal stem cells obtained under conventional culture conditions. The phrase "expression of ITGA11 is low" means that expression of ITGA11 is preferably 10% or less, more preferably 5% or less, further preferably 1% or less and particularly preferably 0.5%, compared to that in the mesenchymal stem cells obtained under conventional culture conditions. The phrase "expression of ITGB5 is low" means that the expression of ITGB5 is 70% or less, more preferably 60% or less, further preferably 50% or less and particularly preferably 40% or less, compared to that in the mesenchymal stem cells obtained under conventional culture conditions. The phrase "expression of ITGB1 is low" means that ITGB1 is expressed preferably 90% or less, more preferably 80% or less, further preferably 75% or less and particularly preferably 70% or less, compared to that in the mesenchymal stem cells obtained under conventional culture conditions. The phrase "expression of ITGBL1 is low" means that expression of ITGBL1 is preferably 70% or less, more preferably 40% or less, further preferably 30% or less and particularly preferably 25% or less, compared to that in the mesenchymal stem cells obtained under conventional culture conditions. The phrase "expression of ITGAV is low" means that expression of ITGAV is preferably 80% or less, more preferably 70% or less, further preferably 60% or less and particularly preferably 35% or less, compared to that in the mesenchymal stem cells obtained under conventional culture conditions. In the mesenchymal stem cells of the present invention, expression of ITGAE, ITGAM, ITGB7 or ITGA5 may be low compared to those in the mesenchymal stem cells obtained under conventional culture conditions.

The phrase "expression of an integrin related gene is high" means that expression of the integrin related gene is preferably 110% or more, more preferably 120% or more, further preferably 130% or more and particularly preferably 150% or more, compared to that in the mesenchymal stem cells obtained under conventional culture conditions. More specifically, the phrase "expression of ITGA10 is high" means that expression of ITGA10 is preferably 200% or more, more preferably 500% or more, further preferably 750% or more and particularly preferably 900% or more, compared to that in the mesenchymal stem cells obtained under conventional culture conditions. The phrase "expression of ITGA7 is high" means that expression of ITGA7 is preferably 150% or more, more preferably 200% or more, further preferably 300% or more and particularly preferably 400% or more, compared to that in the mesenchymal stem cells obtained under conventional culture conditions. The phrase "expression of ITGA9 is high" means that expression of ITGA9 is preferably 130% or more, more preferably 150% or more, further preferably 200% or more and particularly preferably 250% or more, compared to that in the mesenchymal stem cells obtained under conventional culture conditions. The phrase "expression of ITGA4 is high" means that expression of ITGA4 is preferably 120% or more, more preferably 150% or more, further preferably 180% or more and particularly preferably 200% or more, compared to that in the mesenchymal stem cells obtained under conventional culture conditions. In the mesenchymal stem cells of the present invention, expression of ITGA3 or ITGB3 may be high compared to that in the mesenchymal stem cells obtained under conventional culture conditions.

The term "mesenchymal stem cells" in the present invention refers to cells being capable of differentiating into one or more cells, preferably two or more cells, and further preferably three or more cells belonging to the mesenchyme (bone cells, cardiomyocytes, cartilage cells, tendon cells, adipose cells and the like), and capable of proliferating while keeping the capability. The term "mesenchymal stem cells" as used in the present invention refers to cells same as mesenchymal stromal cells and the two are not particularly differentiated. In addition, the term is simply denoted as mesenchymal cells. Examples of tissue containing mesenchymal stem cells include adipose tissue, umbilical cord, bone marrow, umbilical cord blood, endometrial, placenta, amnion, chorion, decidua, dermis, skeletal muscle, periosteum, dental sac, periodontal ligament, dental pulp, and tooth germ. For example, the term "adipose tissue-derived mesenchymal stem cells" refers to mesenchymal stem cells contained in adipose tissues, and may also be referred to as adipose tissue-derived mesenchymal stromal cells. Of these tissues, in view of efficacy for treatment of liver disease and availability and the like, adipose tissue-derived mesenchymal stem cells, umbilical cord-derived mesenchymal stem cells, bone marrow-derived mesenchymal stem cells, placenta-derived mesenchymal stem cells and dental pulp-derived mesenchymal stem cells are preferable; adipose tissue-derived mesenchymal stem cells and umbilical cord-derived mesenchymal stem cells are more preferable; and an adipose tissue-derived mesenchymal stem cells are further preferable.

Mesenchymal stem cells in the present invention may be derived from the same species as or different species from that of a subject to be treated (test subject). Examples of the species of mesenchymal stem cells in the present invention include human, monkey, horse, cattle, sheep, pig, dog, cat, rabbit, mouse and rat, and preferably the mesenchymal stem cells are cells derived from the same species as that of a subject to be treated (test subject). The mesenchymal stem cells in the present invention may be derived from a subject to be treated (test subject); that is, isogenic (autologous) cells, or may be derived from another subject of the same species; that is, may be allogeneic cells. The mesenchymal stem cells are preferably allogeneic cells.

Mesenchymal stem cells are unlikely to cause rejection also in an allogeneic test subject. Therefore, previously prepared donor cells subjected to expansion culture and then cryopreservation can be used as mesenchymal stem cells in the pharmaceutical composition of the present invention. Accordingly, compared with a case in which autologous mesenchymal stem cells are prepared for use, mesenchymal stem cells in the present invention are more preferably allogeneic in view of easy commercialization and ease of obtaining some stable effectiveness.

The term "mesenchymal stem cells" in the present invention refers to any cell population containing mesenchymal stem cells. Such a cell population comprises at least 20% or more, preferably 30%, 40%, 50%, 60%, 70%, 75%, 80%, 85%, 90%, 93%, 96%, 97%, 98% or 99% of mesenchymal stem cells.

The term "adipose tissue" in the present invention refers to tissue containing adipose cells and mesenchymal stem cells including microvascular cells and the like, which can be obtained through surgical resection or suction of subcutaneous fat of mammals, for example. Adipose tissue can be obtained from subcutaneous fat. Adipose tissue is preferably obtained from animals of the same species as that of a test subject to which adipose tissue-derived mesenchymal stem cells are administered as described later. In view of administration to humans, adipose tissue is more preferably human subcutaneous fat. A donor (individual) from which subcutaneous fat is supplied may be alive or dead, however, adipose tissue to be used in the present invention is preferably tissue collected from a living individual. When adipose tissue is collected from an individual, examples of liposuction include PAL (power-assisted) liposuction, elcornia laser liposuction, and body jet liposuction. In view of maintaining cell status, preferably no ultrasonic wave is used.

The umbilical cord in the present invention is a white tubular tissue connecting between the fetus and the placenta, is composed of umbilical cord veins, umbilical cord arteries, mucous connective tissue (Wharton's Jelly), umbilical cord matrix itself, and the like, and is rich in mesenchymal stem cells. The umbilical cord is preferably obtained from animals of the same species as that of a test subject (a subject to which the mesenchymal stem cells are administered) for which the therapeutic agent for diseases of the present invention is used. In view of administration of the mesenchymal stem cells for diseases of the present invention to humans, the umbilical cord is more preferably human umbilical cord.

The term "bone marrow" in the present invention refers to spongy tissue filling the bone lumen and is a hematopoietic organ. Bone marrow aspirate is present in the bone marrow, and cells existing therein are referred to as "bone marrow cells". Bone marrow cells include, in addition to erythrocytes, granulocytes, megakaryocytes, lymphocytes, adipose cells and the like, mesenchymal stem cells, hematopoietic stem cells, vascular endothelial precursor cells, and the like. Bone marrow cells can be collected from human ilia, long bones, or other bones, for example.

The term "mesenchymal stem cells derived from each tissue" in the present invention such as adipose tissue-derived mesenchymal stem cells, umbilical cord-derived mesenchymal stem cells, and bone marrow-derived mesenchymal stem cells refer to any cell population containing mesenchymal stem cells derived from each tissue such as adipose tissue-derived mesenchymal stem cells, umbilical cord-derived mesenchymal stem cells, and bone marrow-derived mesenchymal stem cells, respectively. Such a cell population comprises at least 20% or more, preferably 30%, 40%, 50%, 60%, 70%, 75%, 80%, 85%, 90%, 93%, 96%, 97%, 98% or 99% of mesenchymal stem cells derived from each tissue such as adipose tissue-derived mesenchymal stem cells, umbilical cord-derived mesenchymal stem cells, and bone marrow-derived mesenchymal stem cells.

In the present invention, the mesenchymal stem cells may be characterized in that expression of TF is low, in addition, characterized by, for example, growth characteristics (e.g., population doubling capability or doubling time required from passages to senescence), karyotype analysis (e.g., normal karyotype; maternal or neonatal sequence), surface marker expression as determined by flow cytometry (e.g., FACS analysis), immunohistochemistry and/or immunocytochemistry (e.g., epitope detection), gene expression profiling (e.g., gene chip arrays; polymerase chain reaction such as reverse transcription PCR, real time PCR, and conventional PCR)), miRNA expression profiling, protein arrays, secretion of proteins such as cytokines (e.g., analysis of plasma clotting, ELISA, and cytokine arrays) metabolites (metabolome analysis), other methods known in the art, and the like.

### (Method for preparing mesenchymal stem cells)

A method for preparing low-TF expression mesenchymal stem cells is not particularly limited. Preparation can be carried out as follows: mesenchymal stem cells can be obtained by separating mesenchymal stem cells from a tissue such as an adipose tissue, umbilical cord or bone marrow in accordance with a method known to a person skilled in the art, culturing the mesenchymal stem cells and separating low-TF expression mesenchymal stem cells by a cell sorter using an anti-TF antibody specifically binding to TF or removing High-TF expression cells by use of, e.g., magnetic beads. Alternatively, low-TF expression mesenchymal stem cells can be obtained by inducing them by culture using a specific medium. In the cell population obtained by the induction, preferably 50% or more of the cells constituting the population is low-TF expression cells, more preferably 70% or more thereof is low-TF expression cells; further preferably 80% or more thereof is low-TF expression cells and particularly preferably 90% or more thereof is low-TF expression cells. Most preferably, the cell population is a homogeneous population substantially consisting of low-TF expression cells.

A method for preparing mesenchymal stem cells in which individual integrin related genes (proteins) are expressed at a low level or high level, is not particularly limited; for example, the mesenchymal stem cells can be prepared as follows: the integrin related gene (protein) low-expression cells can be obtained by separating mesenchymal stem cells from a tissue such as an adipose tissue, umbilical cord and bone marrow, in accordance with a method known to a person skilled in the art, culturing them and separating by a cell sorter using an antibody specifically binding to the integrin related protein or removing integrin-related protein high-expression cells by use of, e.g., magnetic beads. Integrin related gene (protein) high-expression cell can be obtained by separating mesenchymal stem cells from a tissue such as an adipose tissue, umbilical cord and bone marrow, in accordance with a method known to a person skilled in the art, culturing them and separating by a cell sorter using an antibody specifically binding to the integrin related protein. Alternatively, integrin related gene (protein) low-expression or high-expression mesenchymal stem cells can be obtained by inducing them by culture using a specific medium. In the cell population obtained by such an induction, preferably 50% or more of the cells constituting the population, more preferably 70% or more, further preferably 80% or more, and particularly preferably 90% or more is the cells exhibiting either one of the features (low-expression or high-expression of an integrin related gene (protein)). The cell population is most preferably a homogeneous cell population substantially consisting of cells having one of the features. A method for preparing low-TF expression mesenchymal stem cells will be specifically described below.

Mesenchymal stem cells can be prepared by a method well-known by persons skilled in the art. A method for preparing adipose tissue-derived mesenchymal stem cells is described below as an example. Adipose tissue-derived mesenchymal stem cells may be obtained, for example, by the production method disclosed in U.S. Patent No. 6,777,231, and can be produced, for example, by a method comprising the following steps (i) to (iii):
(i) step of obtaining a cell suspension by enzymatic digestion of adipose tissue;
(ii) step of precipitating cells for re-suspension of cells in an appropriate medium; and
(iii) step of culturing cells on a solid surface and then removing cells not binding to the solid surface.

The adipose tissue to be used in step (i) is preferably washed. The tissue can be washed with a physiologically compatible physiological saline solution (for example, phosphate buffer saline (PBS)) while vigorously stirring, and then, allowed to precipitate. This is for removing undesired substances (also referred to as debris, for example, damaged tissue and blood such as red blood cells) from the tissue. Accordingly, the wash/precipitation will be repeated until the debris is totally removed from the supernatant. Since the remaining cells are present as clumps different in size, it is preferable that the cell clumps obtained are washed and treated with an enzyme (for example, collagenase, dispase or trypsin) that weakens or destroys intercellular binding in order to mutually dissociate clumps while minimizing damage of the cells themselves. The amount of such an enzyme and a treatment time thereof, which vary depending on the use conditions, are known in this technical field. Cell clumps can be dissociated by using other treatment methods using mechanical stirring, ultrasonic energy and thermal energy in place of or in combination with such an enzymatic treatment. However, in order to minimize cell damage, an enzymatic treatment alone is preferably used. If an enzyme is used, in order to minimize harmful effects to cells, the enzyme is desirably inactivated by use of, e.g., a medium after an appropriate period of time.

The cell suspension obtained in the step (i) contains a slurry or suspension of aggregated cells and various undesirable cells, such as red blood cells, smooth muscle cells, endothelial cells and fibroblasts. The aggregated cells and the undesirable cells may be separated and removed; however since removal can be made by adhesion and washing in the step (iii) described later, separation/removal herein may be skipped. Separation and removal of undesirable cells can be achieved by centrifugation for forcibly separating cells into the supernatant and the sediment. The sediment containing undesirable cells is suspended in a physiologically compatible solvent. The cell suspension may be at a risk of containing red blood cells; however, red blood cells can be selectively removed by adhesion to a solid surface (described later). Because of this, a step of lysing red blood cells is not necessary. As a method for selectively lysing red blood cells, a method known in the art, such as lysis with ammonium chloride through incubation in a hypertonic medium or hypotonic medium, can be used. After lysis, a lysate may be separated from desired cells, for example, by filtration, centrifugal sedimentation or density fractionation.

In the step (ii), to increase purity of mesenchymal stem cells in a cell suspension, washing is carried out once or continuously a plurality of times, centrifugation and resuspension in a medium may be carried out. Other than this, cells may be separated based on a cell surface marker profile or cell size and granularity.

The medium to be used for resuspension is not particularly limited as long as the mesenchymal stem cells can be cultured. Such a medium may be prepared by adding a serum in a basal medium and/or adding at least one serum substitute such as albumin, transferrin, a fatty acid, insulin, sodium selenite, cholesterol, collagen precursor, a trace element, 2-mercaptoethanol and 3'-thiol glycerol. If necessary, substances such as lipids, amino acids, proteins, polysaccharides, vitamins, growth factors, small molecule compounds, antibiotic substances, antioxidants, pyruvic acids, buffers and inorganic salts may be added to such medium.

Examples of the basal medium include IMDM, Medium 199, Eagle's Minimum Essential Medium (EMEM), MEM, MEMα, Dulbecco's modified Eagle's Medium (DMEM), Ham's F12 medium, RPMI 1640 medium, Fischer's medium, MCDB201 medium and a mixed medium of these.

Examples of the serum include, but are not limited to, human serum, fetal calf serum (FBS), bovine serum, calf serum, goat serum, horse serum, porcine serum, sheep serum, rabbit serum and rat serum. If a serum is used, the serum may be added in a ratio of 5 v/v% to 15 v/v%, preferably 10 v/v% relative to the basal medium.

Examples of the fatty acid include, but are not limited to, linoleic acid, oleic acid, linolenic acid, arachidonic acid, myristic acid, palmitoyl acid, palmitic acid and stearic acid. Examples of the lipid include, but are not limited to, phosphatidylserine, phosphatidyl ethanolamine and phosphatidyl choline. Examples of the amino acid include, but are not limited to, L-alanine, L-arginine, L-aspartic acid, L-asparagine, L-cysteine, L-cystine, L-glutamic acid, L-glutamine and L-glycine. Examples of the protein include, but are not limited to, ecotin, reduced glutathione, fibronectin and β2-microglobulin. Examples of the polysaccharide include, but are not limited to, a glycosaminoglycan such as hyaluronic acid and heparan sulfate. Examples of growth factors include, but are not limited to, platelet-derived growth factor (PDGF), basic fibroblast growth factor (bFGF), transforming growth factor β(TGF-β), hepatocyte growth factor (HGF), epidermal growth factor (EGF), connective tissue growth factor (CTGF) and vascular endothelial growth factor (VEGF).

In order to use adipose-derived mesenchymal stem cells obtained in the present invention for cell transplantation, it is preferable to use a xeno-free medium not containing a xenogeneic component(s) such as a serum. As a medium for use in obtaining mesenchymal cells expressing TF at a low level, a commercially available ready-made medium for mesenchymal stem cells (stromal cells) is provided by a manufacturer such as PromoCell GmbH, Lonza, Biological Industries, Veritas, R&D Systems, Corning Inc. and Rohto Pharmaceutical Co., Ltd.

Subsequently, in the step (iii), the cells contained in the cell suspension obtained in the step (ii) are cultured, without separating them, on a solid surface by using an appropriate cell medium as mentioned above at an appropriate cell density and culture conditions. In the present invention, the "solid surface" refers to any material to which the mesenchymal stem cells of the present invention derived from an adipose tissue can bind or adhere. In a particular embodiment, such a material may be a plastic material, the surface of which is treated to promote binding or adhesion of mammalian cells. As a culture vessel having such a solid surface, although the shape thereof is not particularly limited, e.g., a petri dish and a flask, can be preferably used. To remove unbound cells and cell debris, cells are washed after incubation.

In the present invention, cells finally remaining in the state of binding and adhering to the solid surface, can be selected as a cell population of adipose tissue-derived mesenchymal stem cells.

To confirm that the selected cells are the mesenchymal stem cells derived from an adipose tissue according to the present invention, a surface antigen may be analyzed by, e.g., flow cytometry, in accordance with a conventional method. The cells may be examined for an ability to differentiate to various cell lines in accordance with a conventional method.

In the present invention, the mesenchymal stem cells can be prepared as described above. The mesenchymal stem cells can be defined as the cells having the following characteristics other than the characteristics: expression of TF being low; expression of at least one integrin gene selected from the group consisting of ITGA11, ITGA1, ITGB5, ITGBL1, ITGB1 and ITGAV being low; and expression of at least one integrin gene selected from the group consisting of ITGA4, ITGA9, ITGA7 and ITGA10 being high,
(1) adhesive to a plastic material in a culture conditions in a standard medium,
(2) surface antigens CD44, CD73 and CD90 are positive; whereas, surface antigens CD31 and CD45 are negative,
(3) capable of differentiating into osteocytes, adipocytes and chondrocytes in culture conditions.

The mesenchymal stem cells expressing TF protein at a low level can be obtained by selectively separating the cells expressing TF protein at a low level from the mesenchymal stem cells obtained in the step (iii) by an immunological method using, e.g., a cell sorter or magnetic beads. The low-TF expression mesenchymal stem cells can be efficiently obtained by inducing expression of TF at a low level in the mesenchymal stem cells by culturing the mesenchymal stem cells in a predetermined medium which can induce expression of TF at a low level. Further, cells expressing an integrin related gene (protein) at a low level can be obtained by separating the cells from the mesenchymal stem cells obtained in the step (iii) by a cell sorter using an antibody specifically binding to an integrin related protein or by removing cells expressing an integrin related protein at a high level by use of, e.g., magnetic beads. Cells expressing an integrin related gene (protein) at a high level can be obtained by separating the cells from the mesenchymal stem cells obtained in the step (iii) by a cell sorter using an antibody specifically binding to an integrin related protein. Mesenchymal stem cells expressing an integrin related gene (protein) at a low or high level can be obtained by inducing mesenchymal stem cells expressing the integrin related gene (protein) at a low or high level by culture using a predetermined medium. For example, a method for selectively separating low-TF expression cells by an immunological method using a cell sorter will be more specifically described, below.

A cell suspension obtained by treating the mesenchymal stem cells prepared above with, e.g., a trypsin/EDTA solution, is centrifuged (room temperature, 400G, 5 minutes) and the supernatant is removed. To the cells obtained, a staining buffer (1% BSA-PBS) is added and the concentration is controlled to be 1×10⁶ cells/500 µL. After the cell suspension is homogenized with pipetting to obtain a uniform cell concentration, the cell suspension is dispensed to new 1.5 mL micro tubes in a volume of 50 µL per tube. To the cell suspension dispensed, an antibody (PE Mouse anti-human CD142 Clone HTF-1, 550312, manufactured by BD Biosciences) is added. The cell suspension is allowed to react under a light-proof refrigeration condition for 30 minutes to one hour. After washing is carried out three times with a staining buffer (1 mL), 300 µL of PI Buffer (prepared by adding a propidium iodide solution (P4864, manufactured by SIGMA) to the staining buffer so as to obtain a final concentration of 5 to 20 µg/mL) was added and the reaction mixture was sufficiently suspended. The suspension is passed through a tube equipped with a cell strainer and subjected to fluorescence activated cell sorting (FACS) analysis. By the sorting, low-TF expression mesenchymal stem cells can be separated.

### (Cryopreservation of mesenchymal stem cells)

In the present invention, the mesenchymal stem cells may be those obtained by repeatedly and appropriately cryopreserving and thawing, as long as the cells have an effect for treating diseases. In the present invention, cryopreservation can be carried out by suspending mesenchymal stem cells in a cryopreservation solution known to a person skilled in the art and cooling the cells. The suspension can be carried out by removing cells with a remover such as trypsin, transferring the cells in a cryopreservation container, appropriately treating the cells and adding a cryopreservation solution.

The cryopreservation solution may contain DMSO (dimethyl sulfoxide) as a cryoprotective agent; however, DMSO has not only a cytotoxicity and a property for inducing differentiation of the mesenchymal stem cells. Because of this, it is preferable to reduce the content of DMSO. As a substitute for DMSO, glycerol, propylene glycol, polysaccharide and a sugar alcohol are mentioned. If DMSO is used, the concentration of DMSO is 5% to 20%, preferably 5% to 10% and more preferably 10%. Other than DMSO, an additive(s) described in WO2007/058308 may be contained. Such a cryopreservation solution, for example, cryopreservation solutions provided by companies such as Bio Verde Corporation, Nippon Genetics Co., Ltd., REPROCELL, ZENOAQ (Nippon Zenyaku Kogyo Co., Ltd.), COSMO BIO, KOHJIN BIO and Thermo Fisher Scientific, may be used.

When the suspended cells are cryopreserved, it is sufficient that the cells are preserved at a temperature between -80°C and -100°C (for example, -80°C). Cryopreservation can be attained by use of a freezer that can reduce the temperature up to the above temperature. To avoid a rapid temperature change, a cooling rate may be appropriately controlled by use of a program freezer; however, the control means is not limited. The cooling rate may be appropriately selected depending on the components of a cryopreservation solution. Selection can be carried out in accordance with instructions of a manufacturer of a cryopreservation solution.

The preservation period, in other words, upper limit of the period, is not particularly limited as long as the cells cryopreserved in the above conditions and thawed have the equivalent properties as those before cryopreserved. As the upper limit, 1 week or more, 2 weeks or more, 3 weeks or more, 4 weeks or more, 2 months or more, 3 months or more, 4 months or more, 5 months or more, 6 months or more or one year or more is mentioned. Since cell damage can be suppressed by preserving the cells at a lower temperature, the cells may be transferred to a gaseous phase above liquid nitrogen (about - 150°C or lower to -180°C or higher) and preserved there. If the cells are preserved in a gaseous phase above liquid nitrogen, preservation can be made by using a preservation container known to a person skilled in the art. If the cells are preserved for 2 weeks or more, the cells are preferably preserved in a gaseous phase above liquid nitrogen; however, the preservation place is not particularly limited.

The mesenchymal stem cells thawed may be appropriately cultured until a next cryopreservation time. Mesenchymal stem cells are cultured in a medium that can culture the mesenchymal stem cells at a temperature of about 30 to 40°C, preferably about 37°C under the atmosphere containing CO₂; however, the culture conditions are not particularly limited. The concentration of CO₂ is about 2 to 5% and preferably about 5%. In the culture, after cells reach an appropriate confluency in a culture vessel (for example, cells occupy 50% to 80% of a culture vessel), the cells may be removed by a remover such as trypsin, seeded in another culture vessel at an appropriate cell density and continuously cultured. When cells are seeded, a typical cell density is, for example, 100 cells/cm² to 100,000 cells/cm², 500 cells/cm² to 50,000 cells/cm², 1,000 to 10,000 cells/cm² and 2,000 to 10,000 cells/cm². In a particular embodiment, the cell density is 2,000 to 10,000 cells/cm². It is preferable that the period until cells reach an appropriate confluency is controlled to be 3 days to 7 days. During culture, if necessary, the medium may be appropriately exchanged.

Cells cryopreserved can be thawed by a method known to a person skilled in the art, for example, by placing the cells in a thermostat bath or in a hot water bath of 37°C while standing still or shaking.

The state of the mesenchymal stem cells of the present invention is not limited, for example, recovered cells by removing cultured cells and cells freezed in a cryopreservation solution are acceptable. Use of the cells, which were obtained by proliferation in a same culture lot, segmented into small portions and cryopreserved is preferable, because a stable and same function effect can be obtained and handling of the cells is excellent. Cryopreserved mesenchymal stem cells are thawed just before use and suspended in a cryopreservation solution. The suspended mesenchymal stem cells can be directly blended in a solution such as an infusion or a medium, or the cryopreservation solution is centrifugally removed and the resultant cells may be suspended in a solution such as an infusion or a medium. The "infusion" herein refers to a solution to be used in a therapy for a human. Examples of the infusion include, but are not particularly limited to, physiological saline, Japanese Pharmacopoeia physiological saline, a 5% glucose solution, Japanese Pharmacopoeia glucose injection solution, Ringer's solution, Japanese Pharmacopoeia Ringer's solution, Ringer's lactate solution, Ringer's acetate solution, No. 1 solution (starting solution), No. 2 solution (dehydrated replenisher), No. 3 solution (maintaining solution) and No. 4 solution (postoperative recovery solution).

When the mesenchymal stem cells of the present invention are used in a pharmaceutical composition, a pharmaceutically acceptable carrier and additives can be contained other than the above mesenchymal stem cells in accordance with a customary method and depending on the usage or dosage form thereof as long as the effect of the present invention is not damaged. Examples of such carriers and additives include, but are not limited to, isotonizing agents, thickeners, accharides, sugar alcohols, antiseptic agents (preservatives), bactericide agents or antimicrobe agents, pH regulators, stabilizers, chelating agents, oil bases, gel bases, surfactants, suspending agents, binders, excipients, lubricants, disintegrants, blowing agents, fluidizers, dispersants, emulsifiers, buffers, solubilizers, antioxidants, sweeteners, acidifiers, colorants, flavoring agents, essences or refreshing agents. As typical components, for example, the following carriers and additives are mentioned.

Examples of the carrier include an aqueous carrier such as water and hydrous ethanol. Examples of the isotonizing agent (inorganic salt) include sodium chloride, potassium chloride, calcium chloride and magnesium chloride. Examples of the polyhydric alcohol include glycerin, propylene glycol and polyethylene glycol. Examples of the thickener include a carboxyvinyl polymer, hydroxyethyl cellulose, hydroxypropyl methylcellulose, methylcellulose, alginic acid, polyvinyl alcohol (completely or partially saponified), polyvinyl pyrrolidone and macro goal. Examples of the sugar include cyclodextrin and glucose. Examples of the sugar alcohol include xylitol, sorbitol and mannitol (these may be any one of d-form, 1-form and dl-form). Examples of the antiseptic agent, disinfecting agent or antimicrobial agent include dibutylhydroxytoluene, butylhydroxyanisole, an alkyl diaminoethyl glycine hydrochloride, sodium benzoate, ethanol, benzalkonium chloride, benzethonium chloride, chlorhexidine gluconate, chlorobutanol, sorbic acid, potassium sorbate, trometamol, sodium dehydroacetate, methyl parahydroxybenzoate, ethyl parahydroxybenzoate, propyl parahydroxybenzoate, butyl parahydroxybenzoate, oxyquinoline sulfate, phenethyl alcohol, benzyl alcohol, a biguanide compound (more specifically, e.g., polyhexanide hydrochloride (polyhexamethylene biguanide)) and GLOKILL (brand name, manufactured by Rhodia). Examples of the pH modifier include hydrochloric acid, boric acid, aminoethyl sulfonate, epsilon-aminocaproic acid, citric acid, acetic acid, sodium hydroxide, potassium hydroxide, calcium hydroxide, magnesium hydroxide, sodium hydrogen carbonate, sodium carbonate, borax, triethanolamine, monoethanolamine, diisopropanolamine, sulfuric acid, magnesium sulfate, phosphoric acid, polyphosphoric acid, propionic acid, oxalic acid, gluconic acid, fumaric acid, lactic acid, tartaric acid, malic acid, succinic acid, gluconolactone and ammonium acetate. Examples of the stabilizer include dibutylhydroxytoluene, trometamol, sodium formaldehydesulfoxylate (Longarit), tocopherol, sodium pyrosulfite, monoethanolamine, aluminum monostearate, glycerin monostearate, sodium hydrogen sulfite and sodium sulfite. Examples of the oil base include a vegetable oil such as olive oil, corn oil, soybean oil, sesame oil and cotton seed oil, and a medium-chain fatty acid triglyceride. Examples of the aqueous base include, macro goal 400. Examples of the gel base include a carboxyvinyl polymer and gum substance. Examples of the surfactant include polysorbate 80, hardened castor oil, glycerin fatty acid ester and sorbitan sesquioleate. Examples of the suspending agent include white beeswax, a surfactant, gum Arabic, gum Arabic powder, xanthan gum and soy lecithin. Examples of the binder include hydroxyethyl cellulose, hydroxypropyl cellulose, hydroxypropyl methylcellulose, sodium carboxymethylcellulose, polyvinyl pyrrolidone and polyvinyl alcohol. Examples of the excipient include sucrose, lactose, starch, corn starch, crystalline cellulose and light anhydrous silica acid. Examples of the lubricant include sucrose fatty acid ester, magnesium stearate and talc. Examples of the disintegrant include hydroxypropyl cellulose (low degree of substitution), crospovidone and croscarmellose sodium. Examples of the blowing agent include sodium hydrogen carbonate. Examples of the fluidizer include, sodium aluminometasilicate and light anhydrous silicic acid.

The mesenchymal stem cells of the present invention can be provided in various dosage forms such as a solid, a semisolid and a liquid, depending on the purpose. For example, the mesenchymal stem cells of the present invention can be used as a solid agent (e.g., a tablet, a powder, a powdered drug, a granule, a capsule), a semisolid agent [e.g., an ointment (e.g., a hard ointment, a soft ointment), a cream], a liquid agent [e.g., a lotion, an extract, a suspending agent, an emulsion, a syrup, an injection (including an infusion solution, an implant injection, a continuous injection, an injection prepared before use), a dialysis agent, an aerosol agent, a soft capsule, a drink agent], a patch and a poultice. The mesenchymal stem cells of the present invention can be used as a dosage form such as a solution in an oily vehicle or an aqueous vehicle, or an emulsion. Further, the mesenchymal stem cells of the present invention can be applied to an affected area by spraying. The mesenchymal stem cells of the present invention can be used as a dosage form which is designed to forming a gel or sheet after sprayed to an affected area. The mesenchymal stem cells of the present invention can be applied in such a way that the cells form into a sheet-like form or a three dimensional structure, and then, are applied to an affected area.

The mesenchymal stem cells of the present invention can be used by suspending or diluting the cells with an infusion such as physiological saline, Japanese Pharmacopoeia physiological saline, a 5% glucose solution, Japanese Pharmacopoeia glucose injection solution, Ringer's solution, Japanese Pharmacopoeia Ringer's solution, Ringer's lactate solution, Ringer's acetate solution, Ringer's bicarbonate solution, No. 1 solution (starting solution), No. 2 solution (dehydrated replenisher), No. 3 solution (maintaining solution), and No. 4 solution (postoperative recovery solution), or a cell culture medium such as DMEM; preferably with physiological saline, a 5% glucose solution or No. 1 solution (starting solution); and more preferably with a 5% glucose solution or No. 1 solution (starting solution).

If the mesenchymal stem cells of the present invention is used in a liquid, the pH of the liquid is not particularly limited as long as it is within the pharmacologically (pharmaceutically) or physiologically acceptable range. The range is, for example, 2.5 to 9.0, preferably 3.0 to 8.5 and more preferably 3.5 to 8.0.

When the mesenchymal stem cells of the present invention are used in a liquid, the osmotic pressure of the liquid solution is not particularly limited as long as it is within the acceptable range for a living body. The osmotic pressure ratio of the liquid, for example, preferably is within the range 0.7 to 5.0, more preferably 0.8 to 3.0 and further preferably 0.9 to 1.4. The osmotic pressure is controlled by use of, e.g., an inorganic salt, a polyhydric alcohol, a sugar alcohol and/or sugar in accordance with a method known to the art. The osmotic pressure ratio is defined as the ratio of the osmotic pressure of a sample relative to the osmotic pressure of a 0.9w/v% sodium chloride solution (i.e., 286 mOsm) and measured in accordance with the osmometry (freezing point method) described in the 15th amended Japanese Pharmacopoeia. Note that, the standard solution (0.9 w/v% sodium chloride solution) for measuring an osmotic pressure ratio is prepared, after sodium chloride (Japanese Pharmacopoeia standard reagent) is dried at 500 to 650°C for 40 to 50 minutes and cooled in a desiccator (silica gel), by accurately weighing 0.900 g of the sodium chloride obtained, dissolving it in purified water and accurately controlling the volume of the solution to be 100 mL; or a commercially available standard solution for measuring an osmotic pressure ratio (0.9w/v% sodium chloride solution) is used.

Examples of the administration route of the mesenchymal stem cells of the present invention to a subject include oral administration, subcutaneous administration, intramuscular administration, intravenous administration, intra-arterial administration, intraspinal administration, intraperitoneal administration, sublingual administration, transrectal administration, transvaginal administration, intraoculaer administration, transnasal administration, inhalation, transdermal administration, implant and direct administration by spraying an agent and attaching a sheet to the surface of a liver. Since the mesenchymal stem cells of the present invention has been confirmed to have high safety in the case of intravenous administration, intravascular administration such as intravenous administration and intra-arterial administration is preferable and intravenous administration is the most preferable.

The dose of the mesenchymal stem cells of the present invention can vary depending on, e.g., the state (e.g., body weight, age, symptom, physical conditions) of the patient and the dosage form of a pharmaceutical composition of the present invention. In order to produce a sufficient therapeutic effect, it tends to be preferable that the dose is high. In contrast, to suppress the incidence of a side effect, it tends to be preferable that the dose is low. Usually, in the case of administration to an adult, the dose in terms of a number of cells is 1 ×10³ to 1 ×10¹² cells/time, preferably 1 ×10⁴ to 1 ×10¹¹ cells/time, more preferably 1 × 10⁵ to 1 ×10¹⁰ cells/time, and further preferably 5 × 10⁶ to 1 ×10⁹ cells/time. The dose per body weight of a patient is 1 × 10 to 5 × 10¹⁰ cells/kg, preferably 1 × 10² to 5 × 10⁹ cells/kg, more preferably 1 × 10³ to 5 × 10⁸ cells/kg and further preferably 1 × 10⁴ to 5 × 10⁷ cells/kg. Note that, this dose, which is defined as the amount per administration, may be administered a plurality of times or this dose is divided into a plurality of portions and administered.

The mesenchymal stem cells of the present invention may be administered in combination with one or two or more medicinal agents. As the medicinal agent, any medicinal agent(s) may be used. Examples of the medicinal agent include a therapeutic agent for a liver, a therapeutic agent for a heart disease, a therapeutic agent for inflammatory bowel disease, a respiratory medicine, a medicine for the nervous system, a cardiovascular preparation, a brain circulation improver and an immunosuppressive agent.

Examples of a therapeutic agent for liver include a therapeutic agent for hepatitis B (e.g., lamibudine, adefovir, entecavir, tenofovir), interferon preparation (e.g., interferon α, interferon α-2b, interferon β, peginterferon α-2a, peginterferon α-2b), a therapeutic agent for hepatitis C (e.g., ribavirin, Telaprevir, Simeprevir, Vaniprevir, Daclatasvir, Asunaprevir, Sofosbuvir), corticosteroid (e.g., prednisolone, methylprednisolone sodium succinate), an anticoagulant (e.g., dried concentrated human antithrombin III, gabexate mesilate, thrombomodulin α), a detoxicating agent (calcium disodium edetate hydrate, glutathione, dimethycaprole, sodium thiosulfate hydrate, sugammadex sodium), human serum albumin, liver extract, ursodeoxycholic acid, glycyrrhizinic acid, Azathioprine, bezafibrate, amino acids (e.g., glycine, L-cysteine, L-isoleucine, L-leucine, L-valine, L-threonine, L-serine, L-alanine, L-methionine, L-phenylalanine, L-tryptophan, L-ricin, L-histidine, L-arginine and salts of these), vitamin (e.g., tocopherol, flavin adenine dinucleotide, thiamine disulfide phosphate, pyridoxine, cyanocobalamin and salts of these), and antibiotic substances (e.g., sodium sulbactam, sodium cefoperazone, meropenem hydrate, vancomycin hydrochloride).

Examples of the therapeutic agent for a heart disease include an ACE inhibitor, an angiotensin II receptor antagonist, a β blocker, an antithrombocytic agent, warfarin, a calcium antagonist, a nitric acid medicine, a diuretic agent, a HMG-CoA reductase inhibitor and Ancaron.

Examples of the therapeutic agent for inflammatory bowel disease include Sulfasalazine and Mesalazine.

Examples of the respiratory medicine include Dimorpholamine, Doxapram hydrochloride hydrate, sivelestat sodium hydrate, Pirfenidone, pulmonary surfactant and Dornase α.

Examples of the medicine for the nervous system include Edaravone, interferon β-1a, interferon β-1b, fingolimod hydrochloride, Riluzole and taltirelin hydrate.

Examples of the cardiovascular preparation include Hepronicate, Midodrine hydrochloride, Amezinium metilsulfate, Etilefrine hydrochloride and Phenylephrine hydrochloride.

Examples of the brain circulation improver include Ifenprodil tartrate, Nicergoline, Ibudilast, Dihydroergotoxine mesylate, Nizofenone fumarate and Fasudil hydrochloride hydrate.

Examples of the immunosuppressive agent include Cyclosporine, Azathioprine, Mizoribine, Basiliximab, Tacrolimus hydrate, Gusperimus hydrochloride, mycophenolate mofetil and everolimus.

If any one of the aforementioned medicinal agent is administered together with the mesenchymal stem cells of the present invention, the medicinal agent and the mesenchymal stem cells may be stored together with in a same container or separately in different containers. In accordance with, e.g., the type of disease, therapy and state of the patient, the medicinal agent and the mesenchymal stem cells may be administered at the same time or with a certain time interval between administrations.

The mesenchymal stem cells of the present invention can be suitably used in therapy for various diseases; for example, the mesenchymal stem cells are preferably used for visceral diseases such as a heart disease, a stomach/duodenal disease, a small intestine/large intestine disease, a liver disease, a biliary disease, a pancreatic disease, a kidney disease, a lung disease, a mediaphragm disease, a diaphragm disease, a pleural disease, a peritoneal disease, a neurological disease, a central nervous system (CNS) disorder, a peripheral arterial disease and a peripheral vein disease.

Specific examples of the diseases include liver diseases such as autoimmune hepatitis, fulminant hepatitis, chronic hepatitis, viral hepatitis, alcoholic hepatitis, nonalcoholic fatty liver disease (NAFLD), nonalcoholic steatohepatitis (NASH), nonalcoholic fatty liver (NAFL), liver fibrosis, liver cirrhosis, liver cancer, fatty liver, medicinal agent-induced allergic liver disease, hemochromatosis, hemosiderosis, Wilson's disease, primary biliary cirrhosis (PBC), primary sclerosing cholangitis (PSC), biliary atresia, liver abscess, chronic active hepatitis and chronic persistent hepatitis; heart diseases such as myocardial infarction, heart failure, arrhythmia, palpitation, cardiomyopathy, ischemic cardiomyopathy, angina, congenital heart disease, heart valve disease, myocarditis, familial hypertrophic cardiomyopathy, dilated cardiomyopathy, acute coronary syndrome, atherosclerosis and restenosis; stomach/duodenal diseases such as acute gastritis, chronic gastritis, stomach/duodenal ulcer, stomach cancer and duodenal cancer; small intestine/large intestine diseases such as ischemic enteritis, inflammatory bowel disease, ulcerative colitis, Crohn disease, simple ulcer, intestinal Behcet's disease, small intestine cancer and colon cancer; biliary diseases such as acute cholecystitis, acute cholangitis, chronic cholecystitis, cholangiocarcinoma and gallbladder cancer; pancreatic diseases such as acute pancreatitis, chronic pancreatitis and pancreatic cancer; kidney diseases such as acute nephritis, chronic nephritis, acute renal failure and chronic renal failure; pulmonary diseases such as pneumonia, emphysema, pulmonary fibrosis, interstitial pneumonia, idiopathic interstitial pneumonia, desquamative interstitial pneumonia, acute interstitial pneumonia, nonspecific interstitial pneumonia, drug-induced lung disease, eosinophilic lung disease, pulmonary hypertension, pulmonary tuberculosis, pulmonary tuberculosis sequelae, acute respiratory distress syndrome, cystic fibrosis, chronic obstructive pulmonary disease, pulmonary embolism, pulmonary abscess, pneumoconiosis, aspiration pneumonia lung fibrosis, acute upper respiratory tract infection, chronic lower respiratory tract infection, pneumothorax, diseases having damage in the alveolar epithelium, lymphangioleiomyomatosis, lymphatic interstitial pneumonia, alveolar proteinosis and pulmonary Langerhans cell granulomatosis; mediastinal diseases such as mediastinal tumor, mediastinal cystic disease and mediastinitis; diaphragm diseases such as diaphragmatic hernia; pleural diseases such as pleuritis, pyothorax, pleural tumor, cancer pleurisy and pleural mesothelioma; peritoneal diseases such as peritonitis and peritoneal tumor; neurological diseases such as cerebral palsy syndrome including childhood cerebral palsy, aseptic meningitis, Guillain-Barre syndrome, amyotrophic lateral sclerosis (ALS), myasthenia gravis, mononeuropathy, multiple neuropathy, spinal muscular atrophy, spine disorder, acute transverse myelitis, spinal cord infarction (ischemic myelopathy), intracranial tumor and spine tumor; CNS disorder such as Alzheimer disease, cognitive disorder, stroke, multiple sclerosis and Parkinson disease; peripheral arterial diseases such as fibromyal dysplasia, peripheral arterial disease (PAD), obstructive thromboangiitis (Burger's disease) and Kawasaki disease (KD); peripheral vein diseases such as deep vein thrombosis, chronic venous insufficiency, postphlebitic syndrome and superficial venous thrombosis; and immunodeficiency diseases such as graft versus host disease (GVHD), secondary immunodeficiency, primary immunodeficiency disease, B-cell deficiency, T-cell deficiency, combined B- and T-cell deficiency, phagocyte deficiency, complement deficiency in the classical pathway, complement deficiency in the MBL route, complement deficiency in the alternative route, complement regulatory protein deficiency and complement receptor deficiency.

Of these, diseases that have been confirmed to be effectively treated with the mesenchymal stem cells, such as a liver disease, a heart disease, a lung disease, a neurological disease, a peripheral arterial disease, and an immunodeficiency disease, are preferable. In particular, the mesenchymal stem cells can be used more preferably in therapy for diseases such as liver fibrosis, liver cirrhosis, myocardial infarction, heart failure, pulmonary fibrosis, interstitial pneumonia, childhood cerebral palsy, amyotrophic lateral sclerosis (ALS), peripheral arterial disease (PAD) and graft versus host disease (GVHD); and further preferably in therapy for diseases such as liver fibrosis, liver cirrhosis, myocardial infarction, heart failure, pulmonary fibrosis and interstitial pneumonia.

### [Method for treating disease]

According to another aspect of the present invention, the present invention includes a method for treating diseases by using mesenchymal stem cells of which the expression of Tissue Factor (TF) is low; mesenchymal stem cells of which the expression of at least one integrin gene selected from the group consisting of ITGA11, ITGA1, ITGB5, ITGBL1, ITGB1 and ITGAV is low; or mesenchymal stem cells of which the expression of at least one integrin gene selected from the group consisting of ITGA4, ITGA9, ITGA7 and ITGA10 is high. More specifically, according to the present invention, it is possible that the mesenchymal stem cells of the present invention are highly safe and produce therapeutic effects on various diseases by administering the cells to the patients thereof. Note that, to the mesenchymal stem cells for use in the treatment method of the present invention, the same description as in the above section: [mesenchymal stem cells and pharmaceutical composition containing the cells] can be applied.

### [Method for determining for mesenchymal stem cells for use in treatment]

According to another aspect of the present invention, a method for determining whether the mesenchymal stem cells are suitable for treating diseases is included in the present invention. More specifically, the determination method of the present invention includes a step of measuring expression of Tissue Factor (TF) of the mesenchymal stem cells; a step of measuring expression of at least one integrin gene selected from the group consisting of ITGA11, ITGA1, ITGB5, ITGBL1, ITGB1 and ITGAV; and a step of measuring expression of at least one integrin gene selected from the group consisting of ITGA4, ITGA9, ITGA7 and ITGA10. In the above steps, mesenchymal stem cells of which the expression of Tissue Factor (TF) is low; expression of at least one integrin gene selected from the group consisting of ITGA11, ITGA1, ITGB5, ITGBL1, ITGB1 and ITGAV is low; or expression of at least one integrin gene selected from the group consisting of ITGA4, ITGA9, ITGA7 and ITGA10 is high, is determined as being highly safe in treating a disease. Note that, as to the expressions of the Tissue Factor (TF) and various integrins, the same description as in the above section: [Mesenchymal stem cells and pharmaceutical composition containing the cells] can be applied.

### EXAMPLES

Now, the present invention will be more specifically described by way of Examples and Experimental Examples; however, the present invention is not limited by these Examples.

### [Subculture of adipose-derived mesenchymal stem cells]

Adipose tissue-derived mesenchymal stem cells (hereinafter referred to as "ADSC") (product number PT-5006, manufactured by Lonza) were cultured, from P2 to P4, by using a serum-free medium (Rohto Pharmaceutical Co., Ltd.) for mesenchymal stem cells. From P4 to P5, culture was carried out by using a serum-free medium for mesenchymal stem cells or a 10% FBS medium (Minimum Essential Medium α (MEMα)) for 3 days. ADSC attached to a flask were removed by trypsin, transferred to a centrifuge tube and centrifuged at 400 × g for 5 minutes to obtain the cells as a sediment. After the supernatant was removed, a cell cryopreservation solution (STEM-CELLBANKER (ZENOAQ Co., Ltd.)) was add in an appropriate amount to suspend the cells. The cell suspension solution was dispensed in cryotubes and preserved in the freezer at -80°C, transferred to a gaseous phase above liquid nitrogen and continuously preserved as a frozen stock.

### [Examination of therapeutic effect using autoimmune hepatitis model mouse] (Example 1)

To mice (C57BL/6, manufactured by CLEA Japan, Inc., 7 weeks old, male), 20 mg/kg concanavalin A (manufactured by Sigma-Aldrich) was administered in a single dose to induce hepatitis. To the hepatitis-induced mice, the cells of the frozen stock (adipose-derived mesenchymal stem cells cultured in a serum-free medium for mesenchymal stem cells (Rohto Pharmaceutical Co., Ltd.) or adipose-derived mesenchymal stem cells cultured in a 10% FBS medium (MEM)) were intravenously administered in a single dose of 5 × 10⁷ cells/kg. The cells were thawed, washed with MEMα (serum was not added), suspended in HBSS and used for administration. To a non cell-administration group, HBSS alone was administered. In the non cell-administration group and a serum-free medium group for mesenchymal stem cells (a group of mice administered with adipose-derived mesenchymal stem cells cultured in a serum-free medium for mesenchymal stem cells (Rohto Pharmaceutical Co., Ltd.)), all mice survived; whereas, in a 10% FBS medium group (adipose-derived mesenchymal stem cells cultured in a 10% FBS medium (MEMα)), all mice died (Table 1).

**[Table 1]**

| | Dose (cells/kg) | Total number (mice) | Survival number (mice) after 20 h |
|---|---|---|---|
| Non-cell administration group | - | 6 | 6 |
| Serum-free medium group for mesenchymal stem cells | 5 × 10⁷ | 6 | 6 |
| 10% FBS medium group | 5 × 10⁷ | 6 | 0 |

From the above results, it was shown that the 10% FBS medium group (adipose-derived mesenchymal stem cells cultured in a 10% FBS medium (MEMα)) has a problem in safety; whereas, the serum-free medium group for mesenchymal stem cells (the mesenchymal stem cells of the present invention obtained by culture in a serum-free medium for mesenchymal stem cells (Rohto Pharmaceutical Co., Ltd.)) is highly safe.

### [Measurement of size of adipose tissue-derived mesenchymal stem cells] (Example 2)

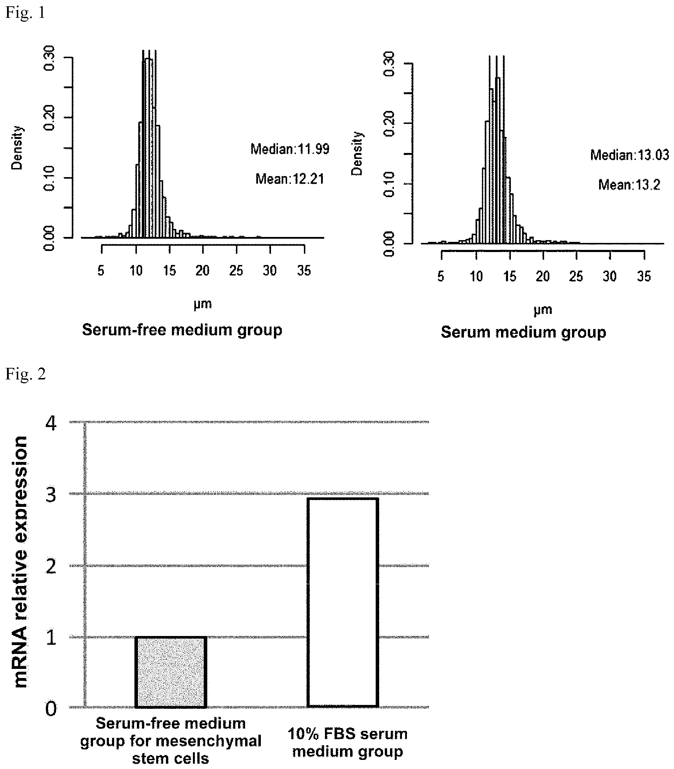

The size of adipose-derived mesenchymal stem cells prepared in the same manner as above was measured by use of CellInsigh CX5 High-Content Screening (HCS) Platform (manufactured by Thermo Fisher Scientific). The cells cultured in a serum-free medium for mesenchymal stem cells (Rohto Pharmaceutical Co., Ltd.) had a mean particle size of 11.99 µm; whereas, the cells cultured in a 10% FBS medium (MEMα) had a mean particle size of 13.03 µm. It was found that the mean particle size of the cells cultured in the serum-free medium for mesenchymal stem cells (Rohto Pharmaceutical Co., Ltd.) is about 10% smaller than that of the cells cultured in the 10% FBS medium (Fig. 1).

### [Effect of administration of cells cultured in 10% FBS medium on mouse survival rate] (Experimental Example 1)

ADSC (product number PT-5006, manufactured by Lonza) were cultured from P2 to P4 in a serum-free medium for mesenchymal stem cells (Rohto Pharmaceutical Co., Ltd.) and from P4 to P5 by using a 10% FBS medium (MEMα) for 3 days. Then, frozen stocks were prepared in the same manner as in the above. When the cells were intravenously administered in a single dose (5 × 10⁷ cells/kg) to normal mice (C57BL/6, manufactured by CLEA Japan, Inc, 9 weeks old, male), all of 6 mice were died in 20 hours after administration. Note that, the cells were thawed, washed with MEMα (serum was not added), suspended in HBSS and used for administration. From the results, it was suggested that a cause of mouse death might be not interaction with concanavalin A induced hepatitis but the cells cultured in a 10% FBS medium (MEMα).

### [TF expression in adipose-derived mesenchymal stem cells] (Example 3)

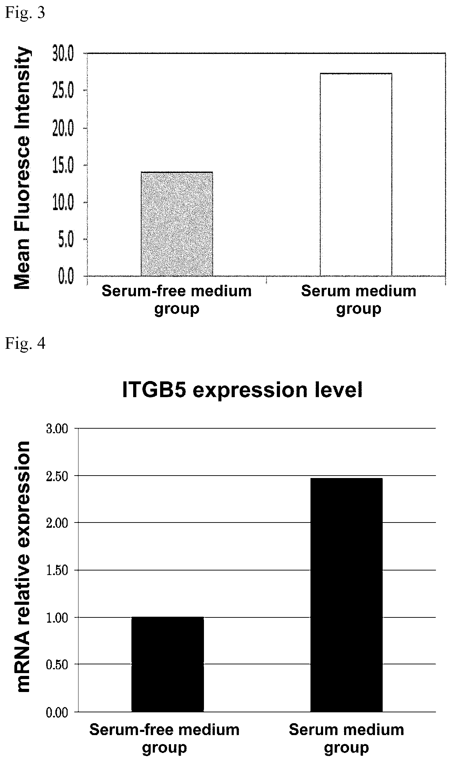

ADSC (product number PT-5006, manufactured by Lonza) were cultured, from P2 to P4, by using a serum-free medium for mesenchymal stem cells (Rohto Pharmaceutical Co., Ltd.) and, from P4 to P5, by using a serum-free medium for mesenchymal stem cells (serum-free medium group) or a 10% FBS medium (MEMα, serum medium group) for 3 days. Then, frozen stocks were prepared in the same manner as in the above. P5 cells of individual stocks were thawed and total RNA was recovered. The expression level of TF mRNA was determined by real time PCR (Fig. 2). Expression at a protein level was detected by flow cytometry (Fig. 3).

In the serum-free medium group compared to the serum medium group, the expression levels of TF mRNA and TF protein were found to be low.

### [Expression 1 of integrin related gene of adipose-derived mesenchymal stem cells] (Example 4)

ADSC (product number PT-5006, manufactured by Lonza) were cultured, from P2 to P4, separately in a serum-free medium for mesenchymal stem cells (serum-free medium group, Rohto Pharmaceutical Co., Ltd.) or a 10% FBS medium (serum medium group). Then, frozen stocks were prepared in the same manner as in the above. P4 cells of individual stocks were thawed, seeded in 6-well plates at a rate of 5,000 cells/cm² and cultured for 3 days separately in the two mediums. The gene expression was analyzed by a microarray method. The results are shown in Table 2.

In the serum-free medium group compared to the serum medium group, it was found that the expression levels of ITGA11, ITGA1, ITGB5, ITGBL1, ITGB1 and ITGAV are significantly low; whereas the expression levels of ITGA4, ITGA9, ITGA7 and ITGA10 are significantly high (Table 2). The numerical values in Table 2 represent relative expression intensities of individual genes (mRNA) expressed by logarithm (base is 2). The "fold change" is a value of serum medium group/serum-free medium group.

**[Table 2]**

| Gene Symbol | Gene Name | Serum-free medium group for mesenchymal stem cells | 10% FBS medium group | Fold change |
|---|---|---|---|---|
| ITGA11 | integrin, alpha 11 | -1.06 | 5.36 | 85.72 |
| ITGA1 | integrin, alpha 1 | -1.92 | 0.99 | 7.55 |
| ITGB5 | integrin, beta 5 | 2.86 | 5.48 | 6.13 |
| ITGBL1 | integrin, beta-like 1 | 3.09 | 5.34 | 4.76 |
| ITGB1 | integrin, beta 1 | 4.15 | 5.62 | 2.77 |
| ITGAV | integrin, alpha V | 4.48 | 5.64 | 2.23 |
| ITGA4 | integrin, alpha 4 | 1.70 | 0.85 | 0.55 |
| ITGA9 | integrin, alpha 9 | 0.03 | -1.32 | 0.39 |
| ITGA7 | integrin, alpha 7 | 6.41 | 4.28 | 0.23 |
| ITGA10 | integrin, alpha 10 | 6.48 | 2.94 | 0.11 |

[Expression 2 of integrin related gene of adipose-derived mesenchymal stem cells] (Example 5)

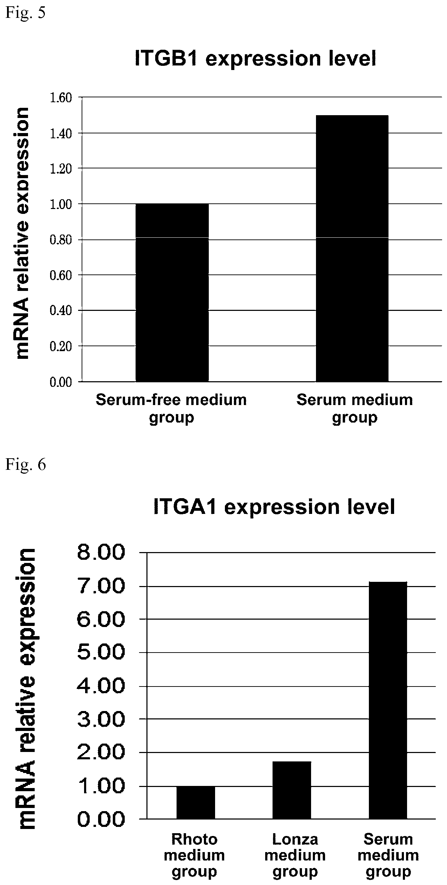

ADSC (product number PT-5006, manufactured by Lonza) were cultured, from P2 to P4, separately in a serum-free medium for mesenchymal stem cells (serum-free medium group, Rohto Pharmaceutical Co., Ltd.) or a 10% FBS medium (serum medium group). Then frozen stocks were prepared in the same manner as in the above. P4 cells of individual stocks were thawed, seeded in 6-well plates in a rate of 5,000 cells/cm² and cultured for 3 days separately in the two mediums. Thereafter, the total RNA of ADSC was recovered and mRNA expression levels of integrin related genes, ITGB5 and ITGB1, were measured by quantitative PCR. The results are shown in Figs. 4 and 5.

As shown in Fig. 4 and Fig. 5, in the serum-free medium group compared to the serum medium group, it was found that the expression levels of ITGB5 and ITGB1 are low.

### [Expression 3 of integrin related gene of adipose-derived mesenchymal stem cells] (Example 6)

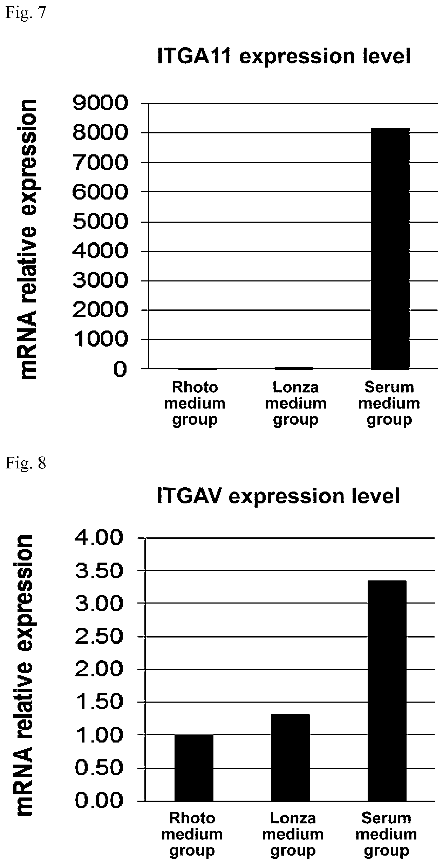

ADSC (product number PT-5006, manufactured by Lonza) were cultured, from P2 to P4, separately in a serum-free medium for mesenchymal stem cells (Rohto Pharmaceutical Co., Ltd., Rohto medium group), a serum-free medium for mesenchymal stem cells (Lonza, Lonza medium group) or a 10% FBS medium (serum medium group). Then, frozen stocks were prepared in the same manner as in the above. P4 cells of individual stocks were thawed, seeded in 6-well plates in a rate of 5,000 cells/cm² and cultured for 3 days separately in the three mediums. Thereafter, total RNA of ADSC was recovered, the mRNA expression levels of integrin related genes of ITGA1, ITGA11, ITGAV and ITGA4 were measured by quantitative PCR. The results are shown in Figs. 6 to 9.

As shown in Figs. 6 to 9, in the serum-free medium group compared to the serum medium group, it was found that the expression levels of ITGA1, ITGA11 and ITGAV are low, and that expression levels of ITGA1, ITGA11 and ITGAV in the Rohto medium group are further lower than those in the Lonza medium group. Also, in the serum-free medium group compared to the serum medium group, it was found that the expression level of ITGA4 is high and that the expression level of ITGA4 is further higher in the Rohto medium group than that in the Lonza medium group.

### [Expression of integrin related gene of adipose-derived mesenchymal stem cells and umbilical cord-derived mesenchymal stem cells] (Example 7)

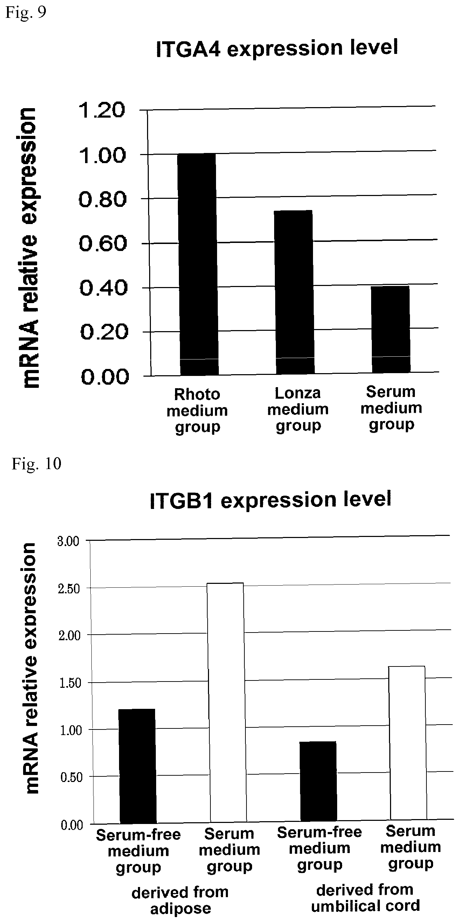

Adipose-derived mesenchymal stem cells (ADSC, product number PT-5006, manufactured by Lonza) and umbilical cord-derived mesenchymal stem cells, from P2 to P4, were separately cultured in a serum-free medium for mesenchymal stem cells (Rohto Pharmaceutical Co., Ltd., serum-free medium group) or a 10% FBS medium (serum medium group). Then, frozen stocks were prepared in the same manner as in the above. P4 cells of individual samples were thawed, seeded in 6-well plates in a rate of 5,000 cells/cm² and cultured for 3 days separately in each medium. Thereafter, the total RNAs of the adipose-derived mesenchymal stem cells and umbilical cord-derived mesenchymal stem cells were recovered and mRNA expression level of an integrin related gene, ITGB1, was measured by quantitative PCR. The results are shown in Fig. 10.

As shown in Fig. 10, it was found that in the umbilical cord-derived mesenchymal stem cells, similarly to the adipose-derived mesenchymal stem cells, the expression level of ITGB1 in the serum-free medium group is low compared to that in the serum medium group.

### [Expression of integrin related gene of adipose-derived mesenchymal stem cells and bone marrow-derived mesenchymal stem cells] (Example 8)

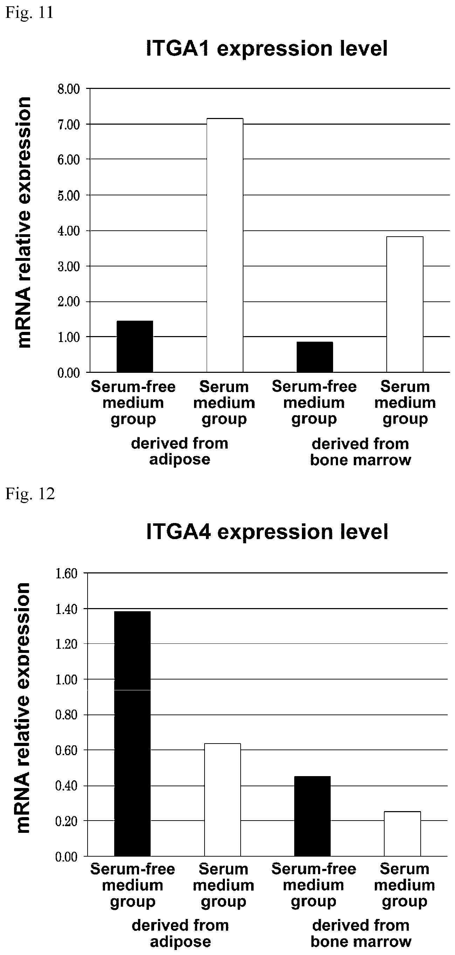

Adipose-derived mesenchymal stem cells (ADSC, product number PT-5006, manufactured by Lonza) and bone marrow-derived mesenchymal stem cells (model number: PT-2501, manufactured by Lonza), from P2 to P4, were separately cultured in a serum-free medium for mesenchymal stem cells (Rohto Pharmaceutical Co., Ltd., serum-free medium group) or a 10% FBS medium (serum medium group). Then, frozen stocks were prepared in the same manner as in the above. P4 cells of individual stocks were thawed, seeded in 6-well plates in a rate of 5,000 cells/cm² and cultured for 3 days separately in each medium. Thereafter, the total RNAs of the adipose-derived mesenchymal stem cells and umbilical cord-derived mesenchymal stem cells were recovered and mRNA expression levels of integrin related genes, ITGA1 and ITGA4, were measured by quantitative PCR. The results are shown in Figs. 11 and 12.

As shown in Fig. 11 and Fig. 12, it was found that in the bone marrow-derived mesenchymal stem cells, similarly to the adipose-derived mesenchymal stem cells, the expression level of ITGA1 is low and the expression level of ITGA4 is high in the serum-free medium group compared to those in the serum medium group.

### [Expression of integrin related gene of adipose-derived mesenchymal stem cells, umbilical cord-derived mesenchymal stem cells and bone marrow-derived mesenchymal stem cells] (Example 9)

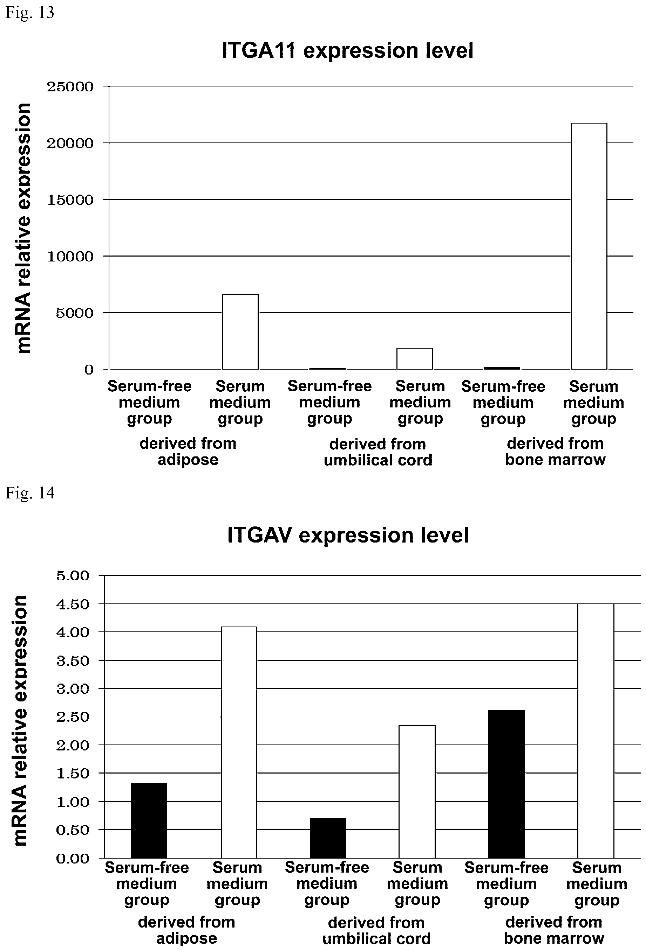

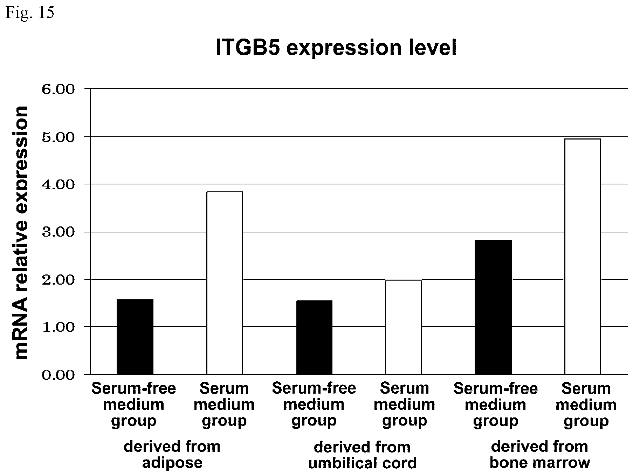

Adipose-derived mesenchymal stem cells (ADSC, product number PT-5006, manufactured by Lonza), umbilical cord-derived mesenchymal stem cells and bone marrow-derived mesenchymal stem cells (model number: PT-2501, manufactured by Lonza), from P2 to P4, were separately cultured in a serum-free medium for mesenchymal stem cells (Rohto Pharmaceutical Co., Ltd., serum-free medium group) or a 10% FBS medium (serum medium group). Then, frozen stocks were prepared in the same manner as in the above. P4 cells of individual stocks were thawed, seeded in 6-well plates in a rate of 5,000 cells/cm² and cultured for 3 days separately in each medium. Thereafter, the total RNA were recovered from each sample of the mesenchymal stem cells and mRNA expression levels of integrin related genes, ITGA11, ITGAV and ITGB5 were measured by quantitative PCR. The results are shown in Figs. 13 to 15.

As shown in Figs. 13 to 15, it was found that in umbilical cord-derived mesenchymal stem cells and bone marrow-derived mesenchymal stem cells, similarly to the adipose-derived mesenchymal stem cells, the expression levels of ITGA11, ITGAV and ITGB5 in the serum-free medium group are low, compared to those of the serum medium group.

### INDUSTRIAL APPLICABILITY

Owing to the present invention, mesenchymal stem cells and a pharmaceutical composition containing the cells are provided. According to the present invention, it is possible to provide highly safe mesenchymal stem cells for use as a medicine.

## Claims

1. Mesenchymal stem cells wherein the expression of Tissue Factor (TF) is low.

2. Mesenchymal stem cells wherein the expression of at least one integrin gene selected from the group consisting of ITGA11, ITGA1, ITGB5, ITGBL1, ITGB1 and ITGAV is low.

3. Mesenchymal stem cells wherein the expression of at least one integrin gene selected from the group consisting of ITGA4, ITGA9, ITGA7 and ITGA10 is high.

4. The mesenchymal stem cells according to any one of claims 1 to 3, wherein the mesenchymal stem cells are allogeneic.

5. The mesenchymal stem cells according to any one of claims 1 to 4, wherein the mesenchymal stem cells are derived from adipose tissue.

6. A pharmaceutical composition comprising the mesenchymal stem cells according to any one of claims 1 to 5.
